# EUROPEAN PATENT SPECIFICATION

(11) **EP 0 728 309 B1**
(45) Date of publication and mention of the grant of the patent: **20.08.1997**
(21) Application number: 95900129.8
(22) Date of filing: 08.11.1994
(51) Int. Cl.: G01N 33/558, G01N 33/53

(54) **ASSAY DEVICES AND THE MANUFACTURE THEREOF**
NACHWEIS VORRICHTUNG UND HERSTELLUNGSVERFAHREN DAFÜR
DISPOSITIFS DE DOSAGE ET LEUR FABRICATION

(30) Priority: 12.11.1993 EP 93309054
(43) Date of publication of application: 28.08.1996
(73) Proprietor: UNIPATH LIMITED, Basingstoke, Hampshire RG24 OPW (GB)
(72) Inventor: MUNDILL, Paul Henry Charles, 44 Ashby Drive, Northampton NN10 9HH (GB); PRIOR, Michael Evans, 330 Newton Road, Northampton NN10 0SY (GB)
(74) Representative: Butler, David John
(86) International application number: EP9403701
(87) International publication number: WO9513542

(56) References cited:
- EP-A- 0 174 247
- EP-A- 0 259 797
- EP-A- 0 492 326
- EP-A- 0 560 410
- WO-A-91/11700
- GB-A- 1 218 749
- DATABASE WPI Week 8849, Derwent Publications Ltd., London, GB; AN 88-349753 & JP,A,63 261 161 (FUJI PHOTO FILM KK) 27 October 1988

## Description

This invention relates to assay devices and to improved ways of manufacturing such devices.

Many assay devices are now available which comprise a porous carrier material, such as a strip, through which applied sample liquid such as urine can permeate and wherein the assay result occurs by means of specific binding of a detectable material in a precisely-defined region (detection zone) of the carrier, such as a narrow line or small dot, containing an immobilized specific binding reagent. Home-use versions of such devices for the analysis of urine, for example in pregnancy tests and ovulation prediction tests, are now widely available commercially. Many such devices are based on the principles of immunochromatography, and typically comprise a hollow casing constructed of plastics material containing a porous assay strip carrying pre-dosed reagents. The reagents within the device can include, for example, one or more reagents labelled with a direct label, such as a dye sol, a metallic (e.g. gold) sol, a non-metallic element (e.g. selenium, carbon) sol, or a coloured latex (polystyrene) microparticle, which are visible to the eye when concentrated in a comparatively small test area of the strip. The user merely needs to apply a urine sample to one part of the casing to initiate the assay. The assay result becomes visible by eye within a few minutes without further action by the user. Examples of such devices are described in EP-A-291194 and EP-A-383619, the disclosures of which are incorporated herein by reference. Sample collection can conveniently be achieved by means of a bibulous member which forms part of the device and which can readily take up sample liquid, eg. from a urine stream. Optionally the bibulous member can protrude from the casing of the device to facilitate sample application.

A particular objective of the invention is to improve the manufacture of assay devices by facilitating the precise locating of a relatively small detection zone within the device. This will have the advantages that less wastage may occur during manufacture by the production of fewer defective devices in which the detection zone is misplaced. Also, the subsequent reading of an assay result in the detection zone is facilitated, because for example an associated optical reading device may not need to "scan" to identify the exact location of the detection zone prior to determining an assay result.

In one embodiment, the present invention provides an assay sample testing device comprising a porous liquid-permeable carrier within a casing, said carrier including a detection zone thereof f in which an assay result is revealed by specific binding of a detectable material directly or indirectly to a binding agent immobilised in said detection zone and said device casing includes internal registration means which cooperatively engage with corresponding registration means associated with said carrier such that said detection zone within said device casing is precisely located in relation to said registration means on said device casing. For example, said internal registration means comprises a peg or the like, engagable with a hole or depression in said carrier, said detection zone being situated at a precise predetermined location on said carrier relative to said hole or depression.

During manufacture of said assay device, said corresponding registration means may be used to facilitate or control accurate formation, e.g. by means of reagent printing techniques, of said detection zone on said carrier. In addition, or alternatively, accurate placement of said carrier within said device casing can be facilitated or controlled by said registration.

EP-A-492326 discloses test strips that are read by insertion into a separate reading device and which are provided with means for positioning the strip in a reading position within the reader.

The invention particulaarly provides a process for the manufacture of an assay device comprising a porous strip within a casing, the strip including at least one detection zone formed by depositing a reagent onto a sheet of porous material prior to subdivision of the sheet into a plurality of identical strips each bearing a portion of the printed reagent, wherein:
a) prior to reagent deposition, the sheet of porous material is provided with a plurality of holes or depressions, at least one hole or depression lying in each region of the sheet which will form an individual strip on subdivision of the sheet;
b) during reagent deposition, the sheet is held by means of the holes or depressions in registration with a printing means such that deposition of the reagent on the sheet is inn a predetermined position relative to the holes or indentations; and
c) following subdivision of the sheet, the assay device is assembled by locating an individual strip within a casing by means of cooperative engagement between said at least one hole or depression and locating means within said casing.

The holes or indentations preferably lie along the longitudinal periphery of the sheet. Reagent deposition can involve printing reagent in one or more lines substantially parallel to an array of said holes or indentations.

A benefit of providing an internal registration system which ensures precise location of the detection zone within the test device, is that automated manufacture and quality control of the testing devices can be facilitated. If the testing devices are intended to be disposable after individual use, they may need to be manufactured in large numbers at low cost. Internal registration can facilitate automated manufacture and high throughput. Other zones requiring critical placement, such as a control zone, can also be located more precisely if desired by means of the invention.

By way of example only, an assay device in accordance with the invention will now be described with reference to the accompanying drawings, of which:
Figure 1 shows a general view of a sheet of carrier material, e.g. nitrocellulose paper, during the course of reagent deposition on the sheet and subdivision of the sheet into assay strips.
Figure 2 shows an "exploded" view of an assay device of the invention incorporating an assay strip made as shown in Figure 1.

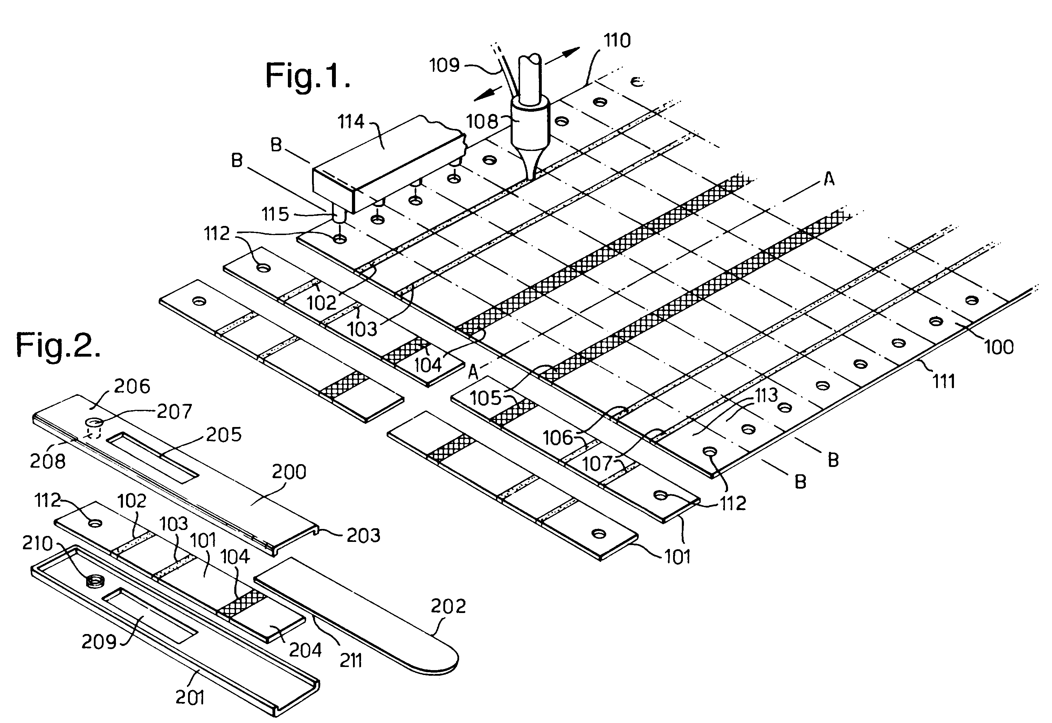

Referring to Figure 1, the sheet 100 of porous carrier material is intended to be divided into a plurality of identical assay strips 101 by cutting along central axis A-A and the lateral axes B-B.

Parallel lines (102-107) of assay reagents are placed on sheet 100 prior to sub-division. For the purposes of example only, the reagents involved are a first immobilised antibody in lines 102 and 107, and a second different immobilised antibody in lines 103 and 106. Reagent deposition can be by means of a "pen" 108 or the like operated on a computer-controlled "x-y" plotting mechanism (not shown) and fed with appropriate buffered reagent solution via a metered flexible tube 109. If the material of sheet 100 is nitrocellulose, reagents such as antibodies or antigens can be immobilised by simple direct application onto the nitrocellulose, followed by blocking of the sheet material with albumen or polyvinyl alcohol. Following reagent deposition and blocking, two lines 104 and 105 of mobile labelled reagent, such as antigen (e.g. E3G, LH) labelled for example with a particulate direct label such as coloured latex, can be deposited. This deposition can be for example by means of an other pen (not shown). Alternatively, the labelled reagent(s) can be held in a separate porous pad or the like, rather than being applied directly to the test strip material.

In order to achieve precise location of the reagent-containing lines, each longitudinal periphery 110, 111 of sheet 100 is pierced with a plurality of identical small holes 112 each one being situated within the width of a designated strip 113. Holes 112 are made in sheet 100 prior to the deposition of any reagents. The untreated sheet is located on a frame (not shown) or similar operating surface by means of a bar 114 pressed downwardly onto each lateral periphery of the sheet. Only one of these bars is (partially) shown. Each bar has a plurality of downwardly projecting pegs 115, each of which locates precisely into one of the holes 112. The tracking of the reagent-depositing pen 108 is registered with the position of the bars holding the sheet, and accordingly the reagent deposition is made in a predetermined precise line relative to the perforations in the sheet.

Following all necessary reagent depositions and other treatments of the sheet, the sheet is subdivided by cutting means (not shown) into individual identical strips 101. Each individual strip therefore contains one locating hole 112 with two reagent-containing lines or reaction zones (e.g. 102 and 103) located relative to hole 112 in precise predetermined positions extending across the width of each strip. At a location remote from hole 112 is a region (e.g. 104) of the strip bearing the mobile labelled reagent. The exact position of the labelled reagent relative to the hole is not critical.

Referring to Figure 2, an assay device of the invention comprises a plastics casing having upper and lower halves 200 and 201 adapted to contain the assay strip 101 and also an optional bibulous sample receiving member 202 which can extend out of one end 203 of the assembled casing. In the assembled device as shown the bibulous receiving member 202 overlaps the end 204 of the assay strip adjacent to the deposited labelled reagent. The upper half 200 of the casing includes an observation window 205 through which both detection zones 102 and 103 can be observed from outside the casing. On the inside of the upper half of the casing is a downwardly extending pin or peg 206. The diameter of the downwardly extending pin or peg 206 matches that of the hole 112 in the assay strip 101, so that the strip can be positively located within the assembled device on the peg.

Lower half 201 of the casing also includes a result observation window 207 which, in the assembled device lies, directly opposite to the result window 205 in the upper half of the casing. Lower half of the casing also contains means, such as a depression 208, which can accommodate the bottom end of the pin or peg 206 when the two halves of the casing are placed together to make an enclosure.

In the assembled device, the act of enclosing the strip and bibulous member between the upper and lower halves of the casing causes the overlapping portions 204 and 209 of the strip and bibulous member to be crimped together to provide a good moisture-conductive junction.

## Claims

1. An assay sample testing device comprising a hollow casing and a porous liquid-permeable carrier contained within said casing, said carrier including a comparatively small detection zone such as a narrow line or small dot in which detection zone an assay result is revealed by specific binding of a detectable material directly or indirectly to a binding agent immobilised in said detection zone, characterised in that said hollow casing includes internal registration means which during manufacture of said device cooperatively engages with corresponding registration means associated with said carrier such that said detection zone is precisely located within said hollow casing in relation to said internal registration means.

2. A device according to claim 1, characterised in that said internal registration means comprises a peg or the like, engagable with a hole or depression in said carrier, said detection zone being situated at a precise predetermined location on said carrier relative to said hole or depression.

3. A process for the manufacture of an assay device according to claim 1 or claim 2, during which said corresponding registration means is used to control accurate formation of said detection zone on said carrier, and accurate placement of said carrier within said hollow casing.

4. A process for the manufacture of an assay device comprising a hollow casing and a porous strip contained within said casing, the strip including at least one detection zone such as a narrow line or small dot formed by depositing a reagent onto a sheet of porous material prior to subdivision of said sheet into a plurality of identical strips each bearing a portion of said printed reagent, characterised in that
a) prior to reagent deposition, said sheet of porous material is provided with a plurality of holes or depressions, at least one hole or depression lying in each region of said sheet which will form an individual strip on subdivision of said sheet;
b) during reagent deposition, said sheet is held by means of the holes or depressions in registration with a printing means such that deposition of said reagent on said sheet is in a predetermined position relative to said holes or indentations; and
c) following subdivision of said sheet, said assay device is assembled by locating an individual strip within said hollow casing by means of cooperative engagement between said at least one hole or depression and locating means within said hollow casing.

5. A process according to claim 4, characterised in that said holes or indentations lie along the longitudinal periphery of said sheet.

6. A process according to claim 4 or claim 5, characterised in that reagent deposition involves printing said reagent in one or more lines substantially parallel to an array of said holes or indentations.

7. A process according to any one of claims 4 to 6, characterised in that said casing is provided with an observation window through which said detection zone can be observed from outside the casing, location of the strip within said hollow casing ensuring that said detection zone is in a predetermined position relative to said observation window.

8. A process according to any one of claims 4 to 7, characterised in that said strip carries more than one detection zone.

9. A process according to any one of claims 4 to 8, characterised in that said locating means within said hollow casing is a peg.

## Revendications

1. Dispositif d'analyse d'échantillon à doser comprenant une enveloppe creuse et un support poreux perméable aux liquides contenu dans ladite enveloppe, ledit support comportant une zone de détection relativement petite, telle qu'une ligne étroite ou un petit point, zone dans laquelle un résultat de dosage est révélé en reliant de manière spécifique une substance détectable directement ou indirectement à un agent de liaison immobilisé dans ladite zone de détection, caractérisé en ce que ladite enveloppe creuse comporte un moyen de positionnement interne qui, pendant la fabrication dudit dispositif, coopère en venant en engagement avec le moyen de positionnement correspondant associé audit support de telle sorte que ladite zone de détection soit disposée de manière précise dans ladite enveloppe creuse par rapport audit moyen de positionnement interne.

2. Dispositif selon la revendication 1, caractérisé en ce que ledit moyen de positionnement interne comprend un ergot ou analogue, pouvant être engagé dans un trou ou un creux dudit support, ladite zone de détection étant située à un emplacement prédéterminé précis sur ledit support par rapport audit trou ou audit creux.

3. Procédé pour la fabrication d'un dispositif de' dosage selon la revendication 1 ou la revendication 2, pendant lequel ledit moyen de positionnement correspondant est utilisé pour contrôler la formation précise de ladite zone de détection sur ledit support, et la mise en place précise dudit support dans ladite enveloppe creuse.

4. Procédé pour la fabrication d'un dispositif de dosage comprenant une enveloppe creuse et un ruban poreux contenu dans ladite enveloppe, le ruban comportant au moins une zone de détection, telle qu'une ligne droite ou un petit point, formée en déposant un réactif sur une feuille de matériau poreux avant la subdivision de ladite feuille en un ensemble de rubans identiques portant chacun une partie dudit réactif imprimé, caractérisé en ce que
a) avant le dépôt du réactif, ladite feuille de matériau poreux est pourvue d'un ensemble de trous ou de creux, avec au moins un trou ou un creux situé dans chaque région de ladite feuille qui formera un ruban individuel par subdivision de ladite feuille ;
b) pendant le dépôt du réactif, ladite feuille est maintenue au moyen des trous ou des creux en étant positionnée par rapport à un moyen d'impression de telle sorte que le dépôt dudit réactif sur ladite feuille soit dans une position prédéterminée par rapport auxdits trous ou auxdites indentations ; et
c) consécutivement à la subdivision de ladite feuille, ledit dispositif de dosage est assemblé en maintenant en place un ruban individuel dans ladite enveloppe creuse au moyen d'un engagement avec coopération entre ledit ou lesdits trou(s) ou creux et ledit moyen de maintien en place dans ladite enveloppe creuse.

5. Procédé selon la revendication 4, caractérisé en ce que lesdits trous ou lesdites indentations s'étendent le long de la partie périphérique longitudinale de ladite feuille.

6. Procédé selon la revendication 4 ou la revendication 5, caractérisé en ce que le dépôt de réactif comprend l'impression dudit réactif en une ou plusieurs lignes sensiblement parallèles à une rangée desdits trous ou desdites indentations.

7. Procédé selon l'une quelconque des revendications 4 à 6, caractérisé en ce que ladite enveloppe est pourvue d'une fenêtre d'observation à travers laquelle ladite zone de détection peut être observée de l'extérieur de l'enveloppe, le maintien en place du ruban dans ladite enveloppe creuse garantissant que ladite zone de détection est dans une position prédéterminée par rapport à ladite fenêtre d'observation.

8. Procédé selon l'une quelconque des revendications 4 à 7, caractérisé en ce que ledit ruban porte plus d'une zone de détection.

9. Procédé selon l'une quelconque des revendications 4 à 8, caractérisé en ce que ledit moyen de maintien en place dans ladite enveloppe creuse est un ergot.

## Patentansprüche

1. Assayprobentestvorrichtung, umfassend ein hohles Gehäuse und einen in dem Gehäuse enthaltenen porösen, flüssigkeitspermeablen Träger, wobei der Träger eine vergleichsweise kleine Nachweiszone, wie eine schmale Linie oder einen kleinen Punkt, einschließt, in welcher Nachweiszone durch spezifische Bindung eines nachweisbaren Materials direkt oder indirekt an ein in der Nachweiszone immobilisiertes Bindungsreagenz ein Assayergebnis offenbart wird, dadurch gekennzeichnet, daß das hohle Gehäuse eine innenliegende Positionierhilfe einschließt, die während der Herstellung der Vorrichtung in eine entsprechende, dem Träger zugeordnete Positionierhilfe, mit dieser zusammenwirkend, eingreift, so daß die Nachweiszone im hohlen Gehäuse bezüglich der innenliegenden Positionierhilfe genau positioniert ist.

2. Vorrichtung nach Anspruch 1, dadurch gekennzeichnet, daß die innenliegende Positionierhilfe einen Zapfen oder dergleichen umfaßt, der mit einem Loch oder einer Vertiefung im Träger in Eingriff gebracht werden kann, wobei die Nachweiszone bezüglich des Lochs oder der Vertiefung an einer genau vorherbestimmten Position auf dem Träger angeordnet ist.

3. Verfahren zur Herstellung einer Assayvorrichtung nach Anspruch 1 oder Anspruch 2, in dem die entsprechende Positionierhilfe zur Steuerung der genauen Ausbildung der Nachweiszone auf dem Träger und zur genauen Plazierung des Trägers im hohlen Gehäuse herangezogen wird.

4. Verfahren zur Herstellung einer ein hohles Gehäuse und einen im Gehäuse enthaltenen porösen Streifen umfassenden Assayvorrichtung, wobei der Streifen mindestens eine Nachweiszone, wie eine schmale Linie oder einen kleinen Punkt, einschließt, die durch Auftragen eines Reagenzes auf einen Bogen porösen Materials ausgebildet wird, bevor der Bogen in eine Mehrzahl identischer Streifen unterteilt wird, wovon jeder eine Portion des gedruckten Reagenzes trägt, dadurch gekennzeichnet, daß
a) vor dem Reagenzienauftrag der Bogen porösen Materials mit einer Mehrzahl von Löchern oder Vertiefungen versehen wird, wobei mindestens ein Loch oder eine Vertiefung in jeder Region des Bogens liegt, die bei Unterteilung des Bogens einen einzelnen Streifen bildet;
b) während des Reagenzienauftrags der Bogen mittels der Löcher oder Vertiefungen zu einer Druckvorrichtung positioniert gehalten wird, so daß der Auftrag des Reagenzes auf den Bogen in vorbestimmter Position bezüglich der Löcher oder Vertiefungen geschieht; und
c) im Anschluß an die Unterteilung des Bogens die Assayvorrichtung zusammengefügt wird, indem ein einzelner Streifen mittels zusammenwirkenden Eingriffs zwischen dem mindestens einen Loch oder der Vertiefung und einer Positionierhilfe im hohlen Gehäuse positioniert wird.

5. Verfahren nach Anspruch 4, dadurch gekennzeichnet, daß die Löcher oder Vertiefungen entlang der Längsperipherie des Bogens liegen.

6. Verfahren nach Anspruch 4 oder Anspruch 5, dadurch gekennzeichnet, daß sich der Reagenzienauftrag das Aufdrucken des Reagenzes in einer oder mehrerer Linien, die im wesentlichen parallel zu einer Anordnung der Löcher oder Vertiefungen liegen, einschließt.

7. Verfahren nach einem der Ansprüche 4 bis 6, dadurch gekennzeichnet, daß das Gehäuse mit einem Beobachtungsfenster versehen ist, durch das die Nachweiszone von außerhalb des Gehäuses betrachtet werden kann, wobei die Lokalisierung des Streifens im hohlen Gehäuse gewährleistet, daß sich die Nachweiszone bezüglich des Beobachtungsfensters in einer vorherbestimmten Position befindet.

8. Verfahren nach einem der Ansprüche 4 bis 7, dadurch gekennzeichnet, daß der Streifen mehr als eine Nachweiszone trägt.

9. Verfahren nach einem der Ansprüche 4 bis 8, dadurch gekennzeichnet, daß die Positionierhilfe im hohlen Gehäuse ein Zapfen ist.
